# EUROPEAN PATENT APPLICATION

(11) **EP 0 863 214 A2**
(43) Date of publication of application: **09.09.1998**
(21) Application number: 98301255.0
(22) Date of filing: 20.02.1998
(51) Int. Cl.: C12Q 1/68, C12N 5/10

(54) **Reporter gene construct for measuring G protein coupled receptor activation**

(30) Priority: 25.02.1997 US 39011 P
(71) Applicant: SMITHKLINE BEECHAM CORPORATION, Philadelphia Pennsylvania 19103 (US)
(72) Inventor: Lee, Jonathon A., King of Prussia, Pennsylvania 19406 (US)
(74) Representative: Crump, Julian Richard John

(57) **Abstract**

A reporter construct and recombinant cells transfected with this construct are provided which are useful for assaying compounds for the agonist or antagonist activity with respect to cell surface localized receptors. Assays methods using these recombinant cells are also provided.

### Annex to the description

SEQUENCE LISTING

(1) GENERAL INFORMATION:

(i) APPLICANT: SmithKline Beecham Corporation

(ii) TITLE OF INVENTION: Novel Reporter Gene Construct for Measuring
G Protein Coupled Receptor Activation

(iii) NUMBER OF SEQUENCES: 1

(iv) CORRESPONDENCE ADDRESS:

(A) ADDRESSEE: F J Cleveland & Company

(B) STREET: 40/43 Chancery Lane

(C) CITY: London

(D) COUNTY:

(E) COUNTRY: United Kingdom

(F) POST CODE: WC2A 1JQ

(v) COMPUTER READABLE FORM:

(A) MEDIUM TYPE: DISKETTE, 3.5 INCH, 1.44 Mb STORAGE

(B) COMPUTER: IBM 486

(C) OPERATING SYSTEM: WINDOWS FOR WORKGROUPS

(D) SOFTWARE: WORDPERFECT 5.1

(vi) CURRENT APPLICATION DATA:

(A) APPLICATION NUMBER: not yet assigned

(B) FILING DATE: Herewith

(C) CLASSIFICATION:

(vii) PRIOR APPLICATION DATA:

(A) APPLICATION NUMBER: 60/039,011

(B) FILING DATE: 25 February 1997

(viii) ATTORNEY/AGENT INFORMATION:

(A) NAME: CRUMP, Julian Richard John

(B) GENERAL AUTHORISATION NUMBER: 37127

(C) REFERENCE/DOCKET NUMBER: P50625

(ix) TELECOMMUNICATION INFORMATION:

(A) TELEPHONE: +44 171 405 5875

(B) TELEFAX: +44 171 831 0749

(2) INFORMATION FOR SEQ ID NO: 1:

(i) SEQUENCE CHARACTERISTICS:

(A) LENGTH: 393

(B) TYPE: Nucleic Acid

(C) STRANDEDNESS: Single

(D) TOPOLOGY: Linear

(iv) ANTI-SENSE: No

(xi) SEQUENCE DESCRIPTION: SEQ ID NO: 1:

ACGCGTATGC TAAAGGACGG TCACATTGCA CAATCTTAAA TGCTAAAGGA 50

CGTCACATTG CACAATCTTA AATGCTAAAG GACGTCACAT TGCACAATCT 100

TAACTCGAGA CTTCAAGCCC TATTCATCCC ATGGCCGTCA TACTGTGACG 150

TCTTTCAGAG CACTTTGTGA TTGCTCAGTC CTAAGTATAA GCCCTATAAA 200

ATGATGGGCT TTGAAATGCT GGTCAGGGTA GAGTGAGAAG CACCAGCAGG 250

CAGTAACAGC CAACCCTTAG CCATTGCTAA GGGCAGAGAA CTGGTGGAGC 300

CTTTCTCTTA CTCCCAGGAC TTCAGCACCT AAGACAGCTC CAAAACAAAC 350

CAGAACAGTC AGCTCCGGGG GAGCACGACT GGGCGAGAAG CTT 393

## Description

### BACKGROUND OF THE INVENTION

Many medically significant processes are mediated by signal transduction pathways that involve the sequential interaction of plasma membrane receptors (G-protein coupled receptors or GPCRs), with guanine nucleotide exchange proteins (G-proteins), and various cellular effector enzymes. These three basic molecular components generate a wide diversity of signal transduction pathways due to (1) the size and ligand recognition diversity of the GPCR superfamily, (2) the number of heterotrimeric combinations of G-proteins, and (3) the diversity of effector proteins, e.g. phospholipases, adenylyl cyclases, phosphodiesterases, channels, and protein kinases.

The molecular diversity of GPCR-mediated signal transduction pathways complicates the configuration of functional assays. In the past, functional GPCR assays quantitated cAMP by radioimmunoassay, production of radiolabeled inositol phosphates, or changes in intracellular Ca levels. Although these assays provide a direct measure of the intracellular second messenger, they generally require large numbers of cells, the use of radioisotopes, and involve multiple liquid transfer steps. Therefore, as currently formatted, these assays are not generally amenable to automated, high through-put execution.

The development of high through-put functional assays for GPCRs would greatly enhance the ability to discover and develop novel agonists and antagonists to this important superfamily of pharmaceutical targets. One approach for developing a high through-put functional GPCR assay is the use of reporter gene constructs. Reporter gene constructs couple transcriptional enhancers that are regulated by various intracellular second messengers with appropriate promoter and reporter gene elements to produce a surrogate signal transduction system responsive to signaling pathways activated by various hormone receptors (Deschamps, *Science,* 1985 *230*:1174-7; Montminy, *Proc. Nail. Acad Sci USA,* 1986 *83*:6682-6686; Angel, *Cell,* 1987, *49*:729-39 ; Fisch, *Mol*. *Cell Biol,* 1989 *9*:1327-31). With the appropriate choice of transcriptional enhancers, promoters, and reporter genes, non-radiometric functional assays have been configured for Gs coupled GPCRs (Konig, *Mol. Cell. Neurosciences,* 1991 2:331-337; *Chen, Anal. Biochemistry*, 1995 *226*: 349-354) and Gq coupled GPCRs (Weyer, *Receptor and Channels,* 1993 *1*:193-200; Stratowa, *J. Rec, and Signal Transduction Research,* 1995, *15(1-4)*:617-630) that are amenable to high through-put screening technology.

Although conceptually straight-forward, the successful development of a useful reporter gene construct depends on the nature of the transcriptional enhancer and basal promoter in the context of the host cell line used. Thus, a particular cyclic AMP response element (CRE) reporter gene was found to be induced by adenylyl cyclase activation in HEK 293 cells, but was inactive in five other cell lines tested (Chen, *Anal. Biochemistry,* 1995, *226*:349-354). Similarly, a given CRE conferred cAMP induction activity when linked to the SV40 promoter and transfected into HeLa, CV-I and NIH3T3 cells but was inactive when transfected into L, HeLa, KB and A431 cells. Moreover, the same CRE acted as a constitutive transcriptional activator when linked to a c-Ha-ras-1 promoter and could either generate, depending on the cell line used, a constitutively active or inactive reporter construct when coupled to a chicken β-actin promoter (Kanei-Ishii, *Nuc. Acids. Res.,* 1989 *17*:1521-36). These results indicate that the utility of a given reporter gene construct, a specific combination of enhancer and promoter elements, must be determined empirically for a given cellular host.

It has now been found that the incorporation of one or more multiple response elements (MREs) into a DNA construct comprising a vasointestinal peptide (VIP) promoter, a cyclic AMP responsive enhancer element (CRE) and a reporter gene produces a significant increase in reporter gene activity following stimulation of intracellular cAMP levels by direct stimulation of adenylyl cyclase or activation of recombinant or endogenous G-protein coupled receptors. The reporter gene construct specifically monitors protein kinase A activity and is not sensitive to activation of protein kinase C pathways. The construct is useful for monitoring cAMP mediated events in HEK 293, COS-1, COS-7, and CHO cell lines transfected with the reporter gene.

### SUMMARY OF THE INVENTION

An object of the present invention is to provide a novel reporter gene construct comprising at least one multiple response element, a cyclic AMP responsive enhancer element, a vasointestinal promoter and a reporter gene.

In accordance with the present invention, there are provided novel recombinant cells transfected with this construct which are useful for assaying compounds for their agonist or antagonist activity with respect to G protein coupled receptors.

Assay methods employing these recombinant cells are also provided. These methods provide a rapid and sensitive means to identify compounds which interact with, and thereby affect the function of G-protein coupled receptors.

### BRIEF DESCRIPTION OF THE FIGURES

Figure 1 shows a schematic representation of an embodiment of a reporter gene construct of the present invention referred to herein as the MREII VIPII reporter gene. In this embodiment, three parallel IL-6 multiple response elements (MREs; Ray, *Mol. Cell Biol.,* 1989, *9*:5537-5547) are upstream from the cAMP response element (CRE) and the basal promoter of the vasointestinal peptide (VIP) gene.

### DETAILED DESCRIPTION OF THE INVENTION

In accordance with the present invention, there is provided a reporter gene construct which comprises at least one MRE and CRE transcriptional control element, a VIP promoter, and a reporter gene. It has been found that transfection of this construct into cell lines commonly used to express recombinant GPCRs results in significant reporter gene expression following stimulation of intracellular cAMP levels.

For example, transfection of the MREII VIPII construct, as depicted in Figure 1, into Human Embryonic Kidney 293 cells resulted in approximately an 80 fold increase in luciferase activity following stimulation of intracellular cAMP levels with forskolin in the presence of the phophodiesterase inhibitor 3-isobutyl-1-methylxanthine (IBMX) as compared to cells transfected with the reporter gene construct which have not been stimulated. Significant stimulation following transfection with this reporter gene construct was also observed in COS-1, COS-7 and CHO cell lines. The broad host cell range of the construct of the present invention contrasts with results reported for other cAMP responsive reporter genes (Chen, *Anal. Biochemistry,* 1985, *226*:349-354).

It was also found that constructs comprising a single MRE instead of three, provided a much larger increase than what is observed in cells containing constructs with either a CRE or a MRE alone. Cells transfected with a construct having a VIP promoter and either a CRE or MRE were induced <10 fold by cAMP. However, the combination of a single MRE and CRE in the construct increased induction to ∼40 fold. Inclusion of multiple MREs with a single CRE further increased cAMP induction to ∼80 fold. Accordingly, MREs are believed to act synergistically with the CRE to produce a higher activity. The level of induction was not increased by further addition of CREs.

Protein kinase specificity of the reporter gene construct of the present invention was also determined. In these experiments, HEK 293 cells were transfected with MREII VIPII and treated with forskolin (15 µM) or TPA (100 nM) to activate protein kinase A or protein kinase C mediated signal transduction pathways, respectively. Significant activation of MREII VIPII was observed after either a 6 or 24 hour stimulation with forskolin in the presence or absence of the phosphodiesterase inhibitor, IBMX. No significant stimulation was observed following activation of protein kinase C by TPA. Accordingly, MREII VIPII principally monitors protein kinase A signal transduction pathways.

GPCR stimulation of the MREII VIPII construct was also determined. In these experiments wild-type HEK 293 cells or HEK 293 cells stabily expressing the porcine CGRP receptor were transfected with MREII VIPII and incubated with various GPCR agonists or forskolin in the presence of IBMX. Luciferase stimulation was observed upon direct activation of adenylyl cyclase by forskolin or agonist mediated stimulation of Gs coupled endogenous receptors, β-adrenergic, calcitonin, PGE2, VIP, or Gs coupled recombinant porcine CGRP receptor. Gi or Gq coupled endogenous receptors for adenosine, endothelin, carbachol, ATP, and ' thrombin did not stimulate luciferase induction from MREII VIPII. Accordingly, it is believed that MREII VIPII specifically monitors increases in cAMP levels by a Gs/protein kinase A mediated signaling pathway.

As will be obvious to those skilled in the art upon this disclosure, in addition to VIP, CREs used in the construct of the present invention can be from other genes including, but not limited to, somatostatin, proenkephalin, phosphoenolpyruvate carboxykinase, or NGFI-A. In addition, reporter genes other than luciferase such as CAT, alkaline phosphatase, and β-galactosidase can be used.

The present invention also comprises recombinant cells useful for assaying compounds to ascertain their agonist or antagonist activity with respect to receptors of a specific type; wherein said receptors are present on the surface of said cell, and wherein said cell is transformed with a reporter gene construct comprising at least one MRE, a CRE, a VIP promoter, and a reporter gene. To produce stable cell lines, the vector containing this construct will also comprise at least one selectable marker. Selection of appropriate vectors to prepare stable cell lines can be performed routinely by those skilled in the art. In a preferred embodiment, the cells are used to screen compounds to ascertain their agonist or antagonist activity with respect to GPCRs present on the cell surface. Methods to assay compounds to determine their cell receptor agonist or antagonist activity are also provided comprising determining the level of the transcriptional and/or translational products of the reporter gene which is produced when a recombinant cell of the present invention is contacted with media containing a compound to be tested. This level is then compared to the level of transcriptional and/or translational products of the reporter gene which is produced when cells of the recombinant cells are contacted with control media not containing the compound to be tested. Agonists of the cell receptor are identified as compounds which cause an increase in the level of transcriptional and/or translational products of the reporter gene as compared to cells not exposed to the compound. Antagonists of the cell receptor are identified as compounds which cause a decrease in the level of transcriptional and/or translational products of the reporter gene in agonist activated cells, as compared to agonist activated cells not exposed to the compound. Alternatively, levels of transcriptional and/or translational products of the reporter in the presence of a potential agonist or antagonist can be compared in cells expressing the receptor on their surface and cells which do not express the receptors on their surface.

Using the method of the present invention, known agonists of Gs coupled GPCRs were demonstrated to stimulate luciferase expression in a dose dependent manner. In these experiments, HEK 293 cells stabily expressing the porcine CGRP receptor were transfected with MREII VIPII and incubated with the indicated GPCR agonists in the presence of IBMX. The apparent EC50 values derived from stimulation of luciferase expression are similar to values derived from conventional assays. This indicates that the reporter assay of the present invention is quantitative and can be useful in identifying and characterizing GPCR agonists and antagonists.

The following nonlimiting examples are provided to further illustrate the present invention.

### EXAMPLES

### Example 1: Construction of MREII VIPII

Oligonucleotides harboring three parallel copies of the IL-6 MRE (-173 to 142; Ray, *Mol Cell Biol.,* 1990, *10*:5736-5746) and MluI and XhoI sites at the 5' and 3' ends, respectively were subcloned into the luciferase vector pGL3-basic (Promega Corp.). PCR was used to amplify the CRE and basal VIP promoter of the VIP gene (-94 to 152; Tsukada, *J*. *Biol. Chem.,* 1987, *262*:8743-8747) from human genomic DNA and to incorporate XhoI and HindIII sites at the 5' and 3' ends of the fragment respectively. Subsequent subcloning of the VIP CRE and basal promoter into MRE pGL-3 basic resulted in plasmid MREII VIPII. The nucleotide sequence of MREII VIPII between MluI and HindIII (SEQ ID NO:1) is shown below.

ACGCGTATGCTAAAGGACGGTCACATTGCACAATCTTAAATGCTAAAGG
ACGTCACATTGCACAATCTTAAATGCTAAAGGACGTCACATTGCACAAT
CTTAACTCGAGACTTCAAGCCCTATTCATCCCATGGCCGTCATACTGTGA
CGTCTTTCAGAGCACTTTGTGATTGCTCAGTCCTAAGTATAAGCCCTATA
AAATGATGGGCTTTGAAATGCTGGTCAGGGTAGAGTGAGAAGCACCAGC

AGGCAGTAACAGCCAACCCTTAGCCATTGCTAAGGGCAGAGAACTGGTG
GAGCCTTTCTCTTACTCCCAGGACTTCAGCACCTAAGACAGCTCCAAAAC
AAACCAGAACAGTCAGCTCCGGGGGAGCACGACTGGGCGAGAAGCTT

### Example 2: DNA Transfection and Luciferase Assay

*Day* 1: HEK293 cells at a density of 4.5x10⁶/T-75 flask in Earl's Minimum Essential Medium (EMEM) supplemented with 10% fetal bovine serum and 2 mM glutamine were incubated overnight in a humidified tissue culture incubator at 37°C, 5% CO₂.

*Day 2*: MREII VIPII DNA (7.8 µg) was mixed with 780 µl of serum free EMEM to produce Solution A. Lipofectamine (23.1 µl; Gibco-BRL) was mixed with 780 µl of serum EMEM to produce Solution B. Solutions A and B were then mixed gently and incubate at room temperature for 15-45 minutes. Cells were then rinsed with 15 ml of serum free EMEM and 6.25 ml of serum free EMEM was added. Combined solutions (A + B) were overlayed and the flask was placed in a tissue culture incubator for 5 hours. Media was replaced with 15 ml of EMEM/10% serum/ 2 mM glutamine and stored in the tissue culture incubator.

*Day 3*: Ninety-six well tissue culture plates were treated with Matrigel (50 µl/well diluted 1/50 with phosphate buffered saline (PBS); Collaborative Biomedical Products) for > 1 hour. Matrigel solution was then removed and the plate was transfected with HEK 293 cells at a cell density of 2.6 x 10⁴/well in 100 µl of supplemented EMEM. Cells were treated 24 or 48 hours following cell seeding with agonists or agents; forskolin (15 µM), IBMX (60 µM), TPA (100 nM), and the agonists PGE2, VIP or CGRP at concentrations of 0.3, 1, 3, 10, 30, 100 and 300 nM. Cells were then incubated for 6 hours in a humidified tissue culture incubator at 37°C, 5% CO₂. Media was removed and cells were washed with 50 µl of PBS.

Cell lysis solution (50 µl; Promega) supplemented with AEBSF (0.24 mg/ml), pepstatin, leupeptin, and aprotinin (5µg/ml each), and DNAse I (50 µg/ml) was added and extracts were frozen. Ten microliters of thawed extract were added to assay plates along with 50 µl of reconstituted luciferase assay reagent (Promega) using a luminometer autoinjector (Dynex MLX) and samples were read. Luciferase activity is expressed as a fold enhancement over the basal luciferase activity expressed in cells that have not been challenged with agonist, IBMX, TPA, or forskolin.

## Claims

1. A reporter gene construct comprising:
(a) a multiple response element;
(b) a cAMP response element:
(c) a vasointestinal peptide promoter; and
(d) a reporter gene.

2. The reporter construct of claim 1 comprising three multiple response elements.

3. A recombinant cell line transformed with a reporter gene construct comprising:
(a) a multiple response element;
(b) a cAMP response element:
(c) a vasointestinal peptide promoter; and
(d) a reporter gene.

4. A method of identifying agonists or antagonists of G protein coupled receptors comprising
(a) contacting a recombinant cell line of claim 3 with a test compound;
(b) determining a level of transcriptional or translational product of the reporter gene in the recombinant cell line; and
(c) comparing this level to a level of transcriptional or translational product of the reporter gene produced in the recombinant cells of claim 3 contacted with control media not containing the test compound.
